# EUROPEAN PATENT SPECIFICATION

(11) **EP 4 203 870 B1**
(45) Date of publication and mention of the grant of the patent: **29.05.2024**
(21) Application number: 21783080.1
(22) Date of filing: 25.08.2021
(51) Int. Cl.: A61F 5/453, A61F 5/44, A61F 5/442

(54) **APPARATUS AND METHODS FOR RECEIVING DISCHARGED URINE**
VORRICHTUNG UND VERFAHREN ZUR AUFNAHME VON AUSGESCHIEDENEM URIN
APPAREIL ET PROCÉDÉS POUR LA RÉCEPTION D'URINE EXCRÉTÉE

(30) Priority: 28.08.2020 US 202063071438 P
(43) Date of publication of application: 05.07.2023
(73) Proprietor: Purewick Corporation, El Cajon, California 92020 (US)
(72) Inventor: WOLFE, Justin, Lawrenceville, Georgia 30044 (US); JOHANNES, Ashley Marie, Statham, Georgia 30066 (US)
(74) Representative: Hoffmann Eitle
(86) International application number: PCT/US2021/047536
(87) International publication number: WO 2022/046894

(56) References cited:
- WO-A1-2018/144463
- WO-A1-2019/212952

## Description

### BACKGROUND

In various circumstances, a person may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, however, urinary catheters can be uncomfortable, painful, and can lead to complications, such as infections. Additionally, bed pans, which are receptacles used for the toileting of bedridden patients, such as those in a health care facility, are sometimes used. Bed pans, however, can be prone to discomfort, spills, and other hygiene issues.

Males who suffer the most severe consequences of urinary incontinence, such as discomfort, rashes, and sores are typically elderly and often bedbound. They also require continuous assistance to maintain hygiene. Characteristics often found in these patients: they typically lay on their back, the size of the penis often decreases with age, skin rolls containing fat tissue cause the penis to recede, often pointing upward while in a laying position, patients have difficulty reaching the penis and manipulating devices. A urine collection device should be designed with reference to these characteristics. WO 2018/144463 A1 describes an urine collection device according to the preamble of claim 1.

### SUMMARY

Embodiments are directed to urine collection devices and systems suitable for collecting and transporting urine away from the body of a person. person, according to claims 1 and 8, respectively.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is an isometric view of a urine collection device, according to an embodiment.
**FIG. 1B** is a side view of the urine collection device shown in **FIG. 1A****.**
**FIG. 1C** is a rear view of the urine collection device shown in **FIG. 1A****.**
**FIG. 2** is an isometric exploded view of the urine collection device shown in **FIG. 1****.**
**FIG. 3A** is a schematic cross-section view of the urine collection device taken through the section lines 3-3 of **FIG. 1C****.**
**FIG. 3B** is a schematic cross-section view of the urine collection device taken through the section lines 3-3 of **FIG. 1C****.**
**FIG. 4A** is an enlarged sectional view of **FIG. 3A** depicting the air inlet having a valve in a closed state.
**FIG. 4B** is an enlarged sectional view of **FIG. 3A** depicting the air inlet having a valve in an open state.
**FIG. 5** is flow diagram of a method for using a urine collection device.
**FIG. 6** is a block diagram of a system for urine collection, according to an embodiment.

### DETAILED DESCRIPTION

Embodiments are directed to urine collection devices suitable for collecting and transporting urine away from the body of a person. Embodiments of a urine collection device may include a receptacle forming or defining an interior volume and configured to receive a penis of the user. The urine collection device can include an air inlet in fluid communication with the interior volume. For example, the air inlet can be defined or otherwise formed within the receptacle. In some embodiments, the air inlet can be configured to retain a tube coupled to an air supply which supplies air to the interior volume of the receptacle to evaporate or otherwise inhibit the presence of humidity or moisture within the interior volume. For example, in at least one, some, or all of the embodiments disclosed herein, air can be drawn into the interior volume through the air inlet and reduce or eliminate any residual moisture (*e.g*., urine) disposed on or within a fluid permeable layer disposed within the interior volume of the urine collection device. Eliminating moisture on or within the urine collection device can prevent the formation of a rash or other source of patient discomfort, especially at areas of the patient's skin which contact or touch a portion of the urine collection device.

The urine collection device can include an outlet in fluid communication with the interior volume and configured to enable extraction of fluid from the receptacle. For example, the outlet can be defined or otherwise formed within the receptacle. In some embodiments, the outlet can be configured to retain a tube coupled to a vacuum source which extracts urine, air, or a combination thereof from the receptacle by applying a negative pressure to the interior volume of the receptacle. One suitable non-limiting example of a vacuum source that can be used is the DryDoc Vacuum Station, available from PureWick, Inc.

In embodiments, the receptacle can include a rim extending around a periphery of the receptacle. A layer of material (*e.g*., hydrogel) can be disposed on the rim before the receptacle is positioned proximate to one or more regions about the penis of the user. The material can create a fluid tight seal between the skin of the user and the receptacle to prevent urine or other fluids within the receptacle from leaking from the receptacle.

In embodiments, the receptacle can include and/or house a plurality of layers, for example, one or more fluid permeable layers, one or more absorbent layers, one or more hydrophobic layers, one or more wicking layers, one or more fluid impermeable layers, and so on. For example a wicking layer can be positioned within the interior volume to provide an interface with the user's genitals that is substantially moisture free. Additionally, or alternatively, the wicking layer can have hydrophobic attributes that cause the fluid to flow to the outlet.

Although the device can receive a portion of the user's penis within the receptacle, the device can be equally beneficial in collecting urine from a user without requiring a portion of the user's penis to be received within the receptacle. For example, a patient having hidden anatomy, such as a buried penis, can still utilize the device despite having substantially inaccessible anatomy.

In some embodiments, a method for transporting urine away from the body may include positioning a receptacle of a urine collection device to abut a region surrounding a penis of a user. The method can include receiving urine within the receptacle of the urine collection device. The method can also include receiving air within the receptacle through an open valve of the receptacle. The method can also include directing the urine through one or more apertures formed within an interior layer of the receptacle to an outlet within the receptacle of the urine collection device responsive to receiving the air within the receptacle. The method can also include removing the urine from the receptacle through the outlet. For example, the urine can be extracted from the outlet via suction device or other suitable apparatus.

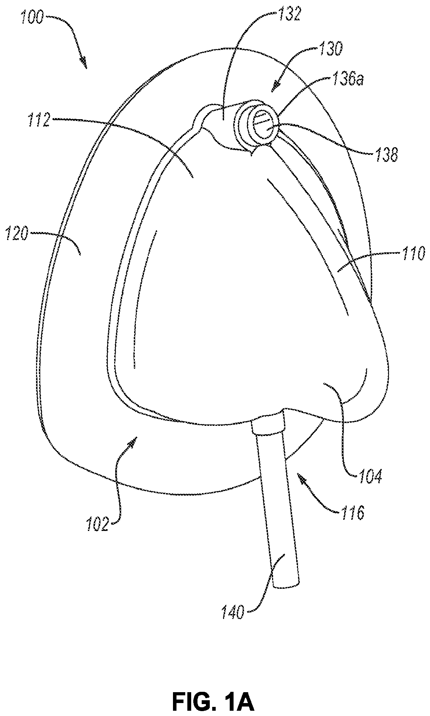

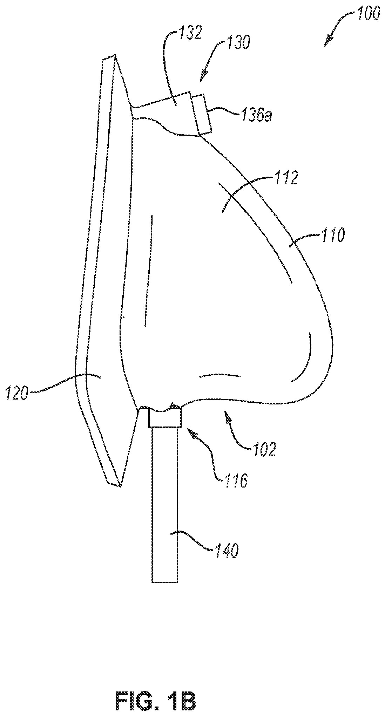

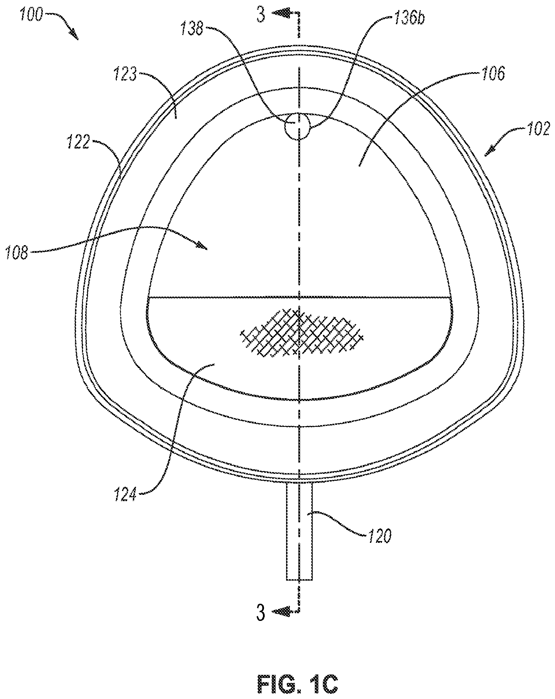

**FIGS. 1A-1C** are isometric, front, side, and rear views, respectively, of a urine collection device 100, according to an embodiment. The urine collection device 100 includes a receptacle 102. The receptacle 102 has semi-elliptically shaped perimeter. An exterior layer 104 of the receptacle 102 forms a generally convex exterior while an interior layer 106 of the receptacle 102 forms a generally concave interior and interior volume 108 (*see* **FIG. 1C**). While the receptacle 102 is depicted as semi-elliptical, other embodiments of the receptacle 102 can be cylindrical, spherical, cubic, a combination thereof, or any other three-dimensional shape capable of defining the interior volume 108.

The exterior layer 104 can be formed of any suitable fluid impermeable materials, such as a fluid impermeable polymer (*e.g.*, silicone, polypropylene, polyethylene, polyethylene terephthalate, polyurethane, a polycarbonate, polyvinyl chloride, latex, silicone, etc.), a metal or alloy layer or film, another suitable material, or combinations thereof.

The exterior layer 104 can define one or more channels 110. In other words, the exterior layer 104 can be molded or otherwise formed to define one or more channels 110 which direct fluid disposed within the urine collection device 100 toward the outlet 116. The channel 110 can protrude from an exterior surface 112 of the exterior layer 104 and form a recess on an interior surface 114 of the exterior layer 104. The channel 110 can be configured to direct or guide the flow of fluids within the receptacle 102 to an outlet 116 defined by the receptacle 102. For example, during use, the receptacle 102 can be positioned such that urine expelled from the patient contacts the interior layer 106 of the receptacle 102 and passes through one or more apertures 118 defined by the interior layer 106 to flow along the channel 110 and into the outlet 116.

In some embodiments, the exterior layer 104 can form a rim 120 that extends around a periphery of the receptacle 102. The rim 120 can form a substantially planar surface 122 which interfaces with a region surrounding the penis of the user. In some embodiments, the planar surface 122 can receive a material 123 which adheres, affixes, or otherwise removably couples the receptacle 102 to the user. The material 123 can be a hydrogel or other suitable adhesive disposed on the planar surface 122 to create a fluid tight seal or barrier to prevent fluids within the receptacle 102 from leaking or seeping onto the user. The fluid tight seal provided by the material 123 can also accommodate or support a negative pressure or suction force applied within the receptacle 102. For example, a vacuum source can be coupled to the outlet 116 to generate a negative pressure within the receptacle 102.

The outlet 116 can form a fluid path or conduit within which fluid (*e.g.,* urine, air, etc.) is extracted from the urine collection device 100. As such, the outlet 116 can be in fluid communication with the interior volume 108, for example, via the one or more apertures 118 formed within the interior layer 106. In some embodiments, the exterior layer 104 can form or define the outlet 116, as illustrated in the embodiment shown in **FIGS. 1A-3B****.** However, in other embodiments, the outlet 116 can be formed by the interior layer 106 or as a separate and distinct component of the urine collection device 100. The outlet 116 can be sized and shaped to receive a tube 140 of a vacuum source (not shown). A cross-sectional shape of the outlet 116 can be circular, oval, rectangular, or any other geometric shape that may conform to the tube 140 of a vacuum source.

In some embodiments, a fluid permeable layer 124 can be disposed within the interior volume 108. The fluid permeable layer 124 can include multiple layers of material, such as, hydrophobic or wicking layers having varied permeable properties. The fluid permeable layer 124 can include any material that can wick fluid. For example, the fluid permeable layer 124 can include fabric, such as a gauze (*e.g.,* a silk, linen, polyester, or cotton gauze), another soft fabric (*e.g.,* jersey knit fabric or the like), or another smooth fabric (*e.g.,* rayon, satin, or the like). In some examples, the fluid permeable layer 124 can include an open cell foam. Forming the fluid permeable layer 124 from gauze, soft fabric, and/or smooth fabric can reduce chafing caused by the urine collection device 100. The fluid permeable layer 124 can additionally or alternatively be formed using one or more layers of polytetrafluoroethylene (PTFE) and/or spun nylon fibers. For example, the fluid permeable layer 124 can include spun nylon fibers with a fabric gauze exterior which contacts a portion of the user's penis while the urine collection device 100 is disposed on the user.

The fluid permeable layer 124 can be coupled to, cover, disposed adjacent to, and/or be in fluid communication with the interior layer 106 and positioned within the interior volume 108 of the receptacle 102. For example, the fluid permeable layer 124 can be adhered to the interior layer 106, such as along a periphery of the interior layer 106. The fluid permeable layer 124 can cover at least a portion of the interior layer 106. For example, the fluid permeable layer 124 can cover a portion of the interior layer 106 that is positioned nearest the outlet 116 of the exterior layer 104, as shown in **FIG. 1C****.** In an embodiment, the fluid permeable layer 124 can cover substantially all of the surface of the interior layer 106 that forms the interior volume 108. The fluid permeable layer 124 can be coupled to the interior layer 106 such that the concaved contour defined by the interior layer 106 of the receptacle 102 is matched by the fluid permeable layer 124. The fluid permeable layer 124 can be removable in some examples by utilizing hook and loop fasteners to allow a replacement layer to be inserted and used within the device 100.

In an embodiment, the fluid permeable layer 124 allows fluid to flow through the material in a first direction, but may prevent fluid from flowing through the material in a second direction. For example, the fluid permeable layer 124 can allow urine to flow through the fluid permeable layer 124 and through the one or more apertures 118 of the interior layer 106 while preventing or inhibiting urine from flowing back out of interior layer 106 and onto the user's skin. In other words, the fluid permeable layer 124 can permit unidirectional fluid flow and thereby obstruct urine from leaking through the permeable layer 124 and contacting the user's skin.

The fluid permeable layer 124 can include permeable material designed to wick or pass fluid therethrough. The permeable properties referred to herein may be wicking, capillary action, diffusion, or other similar properties or processes, and are referred to herein as "permeable" and/or "wicking." Such "wicking" may not include absorption of fluid into the wicking material. Put another way, substantially no absorption of fluid into the material may take place after the material is exposed to the fluid and removed from the fluid for a time. While no absorption is desired, the term "substantially no absorption" may allow for nominal amounts of absorption of fluid into the fluid permeable layer 124 *(e.g.,* absorbency), such as less than about 30 wt% of the dry weight of the fluid permeable layer 124, about 20 wt%, about 10 wt%, about 7 wt%, about 5 wt%, about 3 wt%, about 2 wt%, about 1 wt%, or about 0.5 wt% of the dry weight of the fluid permeable layer 124. The fluid permeable lyer 124l can include natural fibers. In such examples, the fluid permeable layer 124 may have a coating to prevent or limit absorption of fluid into the material, such as a water repellent coating.

In embodiments, the urine collection device 100 can include a second fluid permeable layer 126 adhered or otherwise coupled between the interior layer 106 and an exterior layer 104 of the receptacle 102. For example, a batting material can be positioned between the interior layer 106 and the exterior layer 104 of the receptacle 102.

The fluid permeable layer 124 (*i.e.,* the first fluid permeable layer 124) and/or the second fluid permeable layer 126 can be formed from the same material or different materials. For example, the first and second fluid permeable layers 124, 126 can be made of a material that is urine permeable and has wicking properties. The first and second fluid permeable layers 124, 126 can have a high absorptive rate and a high permeation rate such that urine can be rapidly absorbed and/or transported through the first and second fluid permeable layers 124, 126. In some embodiments, the second fluid permeable layer 126 can be a ribbed knit fabric. In some embodiments, the second fluid permeable layer 126 can include and/or have the moisture-wicking characteristic of gauze, felt, terrycloth, thick tissue paper, and/or a paper towel. In some embodiments, the first fluid permeable layer 124 can be soft and/or minimally abrasive such that the first fluid permeable layer 124 does not irritate the skin of the user. The first fluid permeable layer 124 can wick fluid away from the skin of the user such that moisture within the receptacle 102 is lessened. The second fluid permeable layer 126 can wick fluid away from the interior layer 106 (*e.g.,* the one or more apertures 118 of the interior layer 106). The wicking properties of the first and second fluid permeable layers 124, 126 can help prevent urine from leaking onto, for example, a hospital bed or a user of the urine collection device 100. In some embodiments, the first and second fluid permeable layers 124, 126 can be formed of fine denier polyester fibers coated with a thermoplastic water-based binder system.

Some embodiments of the urine collection device 100 can include the first and second fluid permeable layers 124, 126 while other embodiments may include one or none of the first and second fluid permeable layers 124, 126. For example, an embodiment of the urine collection device 100 can include the first fluid permeable layer 124 coupled to the interior layer 106 and within the interior volume 108. This embodiment may not include the second fluid permeable layer 126 disposed between the interior layer 106 and the exterior layer 104 of the receptacle 102.

In embodiments that include the first fluid permeable layer 124, the first fluid permeable layer 124 can cover at least a portion of an interior surface 128 of the receptacle 102 defined by the interior layer 106. In an embodiment, the first fluid permeable layer 124 can be coupled to the interior layer 106 such that half of the interior surface 128 is covered by the first fluid permeable layer 124. For example, the first fluid permeable layer 124 can cover half of the interior surface 128 positioned nearest the outlet 116 (*e.g.,* the lower half of the interior layer 106 forming the one or more apertures 118). In some embodiments, the first fluid permeable layer 124 can be coupled to and cover a substantial majority of the interior surface 128 of the interior layer 106.

In embodiments that include the second fluid permeable layer 126, the second fluid permeable layer 126 can cover at least a portion of the interior surface 114 of the exterior layer 104. In an embodiment, the second fluid permeable layer 126 can be coupled between the interior layer 106 and the exterior layer 104 such that at least the one or more apertures 118 formed by the interior layer 106 are covered by the second fluid permeable layer 126. For example, the second fluid permeable layer 126 can cover half of the interior surface 114 of the exterior layer 104 nearest the outlet 116 (*e.g.,* the lower half of the exterior layer 104 forming the outlet 116). In some embodiments, the second fluid permeable layer 126 can be disposed between the interior layer 106 and exterior layer 104 to cover a substantial majority of the interior surface 114 of the exterior layer 104.

The urine collection device 100 can include an air inlet 130 in fluid communication with the interior volume 108 of the receptacle 102. In an embodiment, the air inlet 130 can be formed by the interior layer 106 and extend through an aperture 132 within the exterior layer 104. The air inlet 130 can provide air to the interior volume 108 to reduce or eliminate residual moisture within the urine collection device 100. For example, air can be drawn into the interior volume 108 and reduce or eliminate any residual moisture (*e.g.,* urine) disposed within the fluid permeable layer 124 (*i.e.,* the first fluid permeable layer 124) to prevent formation of a rash or other source of patient discomfort.

While the air inlet 130 is illustrated as a singular aperture having a circular cross-sectional shape, the air inlet 130 can be formed of one or more apertures having cross-sectional shapes that resemble a variety of geometric shapes (*e.g.,* circular, oval, square, rectangular, triangular, and so on). In some embodiments, the urine collection device can include a plurality of air inlets, each of the air inlets can be in fluid communication with an interior volume of the urine collection device.

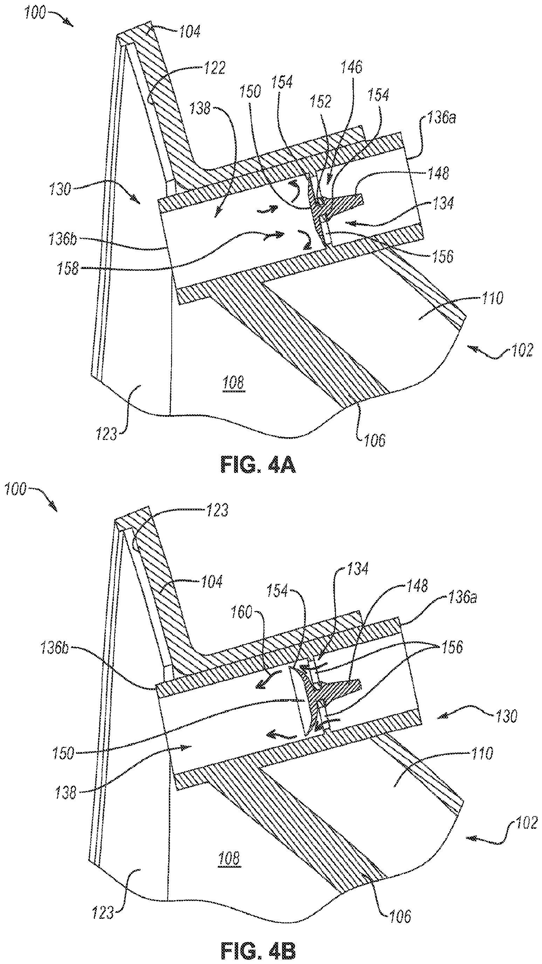

In some embodiments, the air inlet 130 can include an air inlet structure 134 disposed between respective ends 136a, 136b of the air inlet 130 and bisecting an opening 138 of the air inlet 130. The air inlet structure 134 can obstruct objects, such as a tube, from passing through the air inlet 130 and into the interior volume 108. Additionally, or alternatively, the air inlet structure 134 can retain a valve within the opening 138 of the air inlet 130. The air inlet structure 134 and an example valve are described in greater detail with respect to **FIGS. 4A** and **4B****.**

The receptacle 102 can be inclined or otherwise positioned on a user such that the outlet 116 is at a lower elevation or height than the rest of the receptacle 102. Thus, gravitational force can also drive urine through the one or more apertures 118 and into the channel 110. Additionally or alternatively, a vacuum source (not shown) can be coupled to the outlet 116, for example, by disposing the tube 140 of the vacuum source within the outlet 116. The vacuum source can generate or create negative pressure within the receptacle 102 to cause fluids disposed within the receptacle 102 to exit through the outlet 116. The vacuum source can simultaneously cause air to be drawn into the receptacle 102 through the air inlet 130.

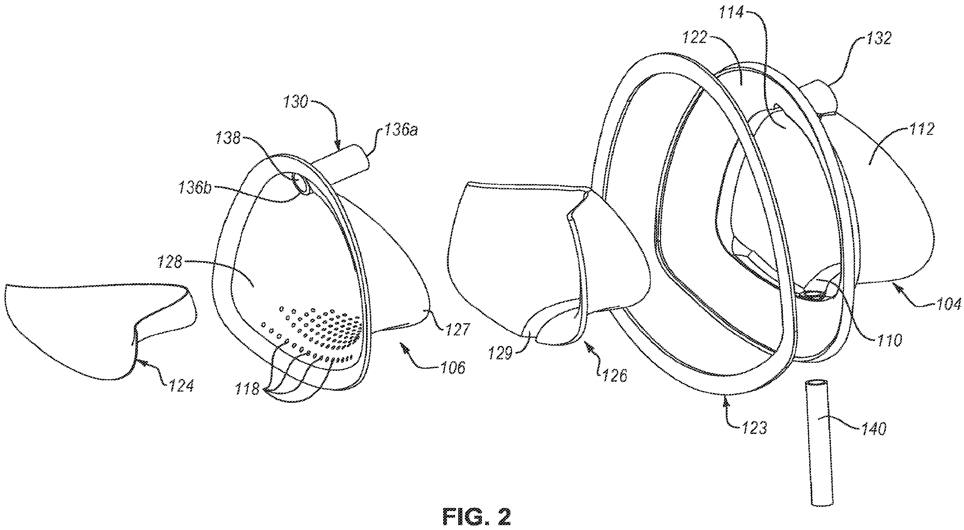

**FIG. 2** shows an exploded isometric view of the urine collection device 100 shown in **FIGS. 1A-1C****.** The urine detection device 100 includes a receptacle 102 formed of the exterior layer 104 and the interior layer 106. The exterior layer 104 and the interior layer 106 can be adhered or otherwise affixed to one another, for example, by an adhesive or other coupling mechanism. In embodiments, the second fluid permeable layer 126 can be adhered or otherwise disposed between the interior layer 106 and an exterior layer 104 of the receptacle 102. The second fluid permeable layer 126 can be a batting material or other wicking material which enables fluid (*e.g.,* urine, air, etc.) to be passed to the one or more channels 110 and/or the outlet 116. In some embodiments, the second fluid permeable layer 126 can define one or more protruding portions 129 which at least partially extend into or are otherwise received by the one or more channels 110. In embodiments, a portion of the second fluid permeable layer 126 can be disposed within a portion of the channel 110.

In embodiments, such as the embodiment shown in **FIG. 2****,** one or both of the first and second fluid permeable layers 124, 126 can conform to the curved surfaces formed by the exterior and interior layers 104, 106. For example, the second fluid permeable layer 126 can conform to the concave interior surface 114 of the exterior layer 104 and the convex exterior surface 127 of the interior layer 106. Similarly, the first fluid permeable layer 124 can conform to the concave interior surface 128 of the interior layer 106, according to at least one embodiment.

The one or more apertures 118 can be formed within the interior layer 106 and can be positioned at any location on the interior layer 106. For example, as illustrated in **FIG. 2****,** the one or more apertures 118 can be formed proximal to the outlet 116 and one or more channels 110 of the exterior layer 104. In other words, the one or more apertures 118 can be formed within regions of the interior layer 106 that are likely to be exposed to urine when the user urinates into the urine collection device 100. In embodiments, the one or more apertures 118 can be evenly distributed throughout an entirety of the interior layer 106 such that urine can pass through the interior layer 106 at substantially any location at the interior surface 128 of the interior layer 106. In some embodiments, as shown in **FIG. 2****,** the one or more apertures 118 can be evenly distributed throughout a particular region or set of particular regions of the interior layer 106 (*e.g.,* the regions abutting the channel 110 and/or outlet 116). The respective diameters of each of the one or more apertures 118 can be uniform or vary relative to the position and quantity of apertures 118. For example, a urine collection device 100 having relatively few apertures 118 may include apertures 118 having relatively large diameters while a urine collection device 100 having relatively numerous apertures 118 may include apertures 118 having relatively small diameters. In other embodiments, the one or more apertures 118 may be non-uniformly distributed.

In embodiments, the first fluid permeable layer 124 can be adhered or otherwise affixed to the interior layer. The first fluid permeable layer 124 can enable fluid (*e.g.,* urine, air, etc.) to be passed to the one or more apertures 118 formed within the interior layer 106. In some embodiments, the first fluid permeable layer 124 can be non-abrasive to mitigate or prevent irritation to any portion of the patient's skin which contacts the first fluid permeable layer 124.

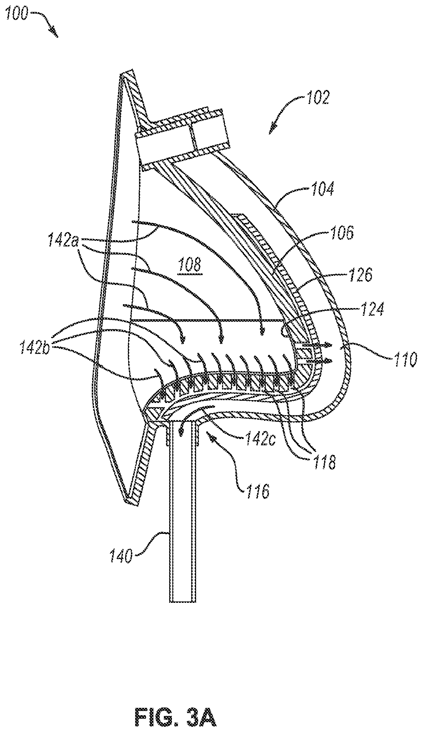

**FIG. 3A** is a section view of the urine collection device 100 taken through the section lines 3-3 of **FIG. 1C**, according to an embodiment. More specifically, **FIG. 3A** illustrates an example of urine flow through the device 100 when a user expels urine into the interior volume 108 of the receptacle 102. As shown by the flow lines 142a, the penis can be at least partially positioned within the interior volume 108 of the receptacle 102 such that urine is expelled directly into the interior volume 108. There may be circumstances in which the penis of the user is in fluid communication with the interior volume 108 but not disposed within the interior volume 108, for example, the user's penis may be receded or buried. In these circumstances, the urine collection device 100 can be positioned over the penis such that urine expelled by the penis will be received within the receptacle 102 of the urine collection device 100.

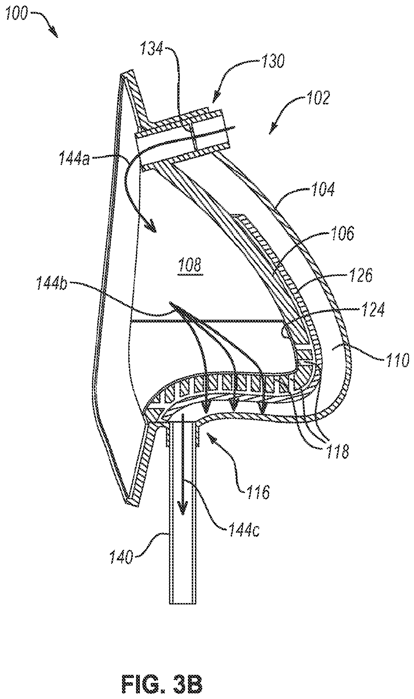

As illustrated by flow lines 142b, the urine disposed within the interior volume 108 flows through the first fluid permeable layer 124 and through the one or more apertures 118 within the interior layer 106. The one or more apertures 118 can be formed at any location on the interior layer 106. For example, as illustrated in **FIGS. 3A** and **3B****,** the one or more apertures 118 can be located proximal to the outlet 116 and channel 110 of the exterior layer 104. In embodiments, the one or more apertures 118 can be evenly distributed throughout an entirety of the interior layer 106 such that urine can pass through the interior layer 106 at substantially any location at the interior surface 128 of the interior layer 106. In some embodiments, the one or more apertures 118 can be evenly distributed throughout a particular region or set of particular regions of the interior layer 106 (*e.g.,* the regions abutting the channel 110 and/or outlet 116) such that urine can pass through the interior layer 106 at the regions of the interior layer 106.

The quantity of the one or more apertures 118 formed within the interior layer 106 can vary from one embodiment to another. For example, some embodiments of the urine collection device 100 may include dozens of apertures 118 formed within the interior layer 106 while other embodiments of the urine collection device 100 may include relatively few apertures 118 formed within the interior layer 106. In embodiments, each of the one or more apertures 118 can define a substantially consistent diameter. In other embodiments, the respective diameters of each of the apertures 118 can vary such that some of the apertures 118 have a diameter that is relatively greater than the diameter of other apertures 118. For example, the one or more apertures 118 positioned nearest the channel 110 and outlet 116 can have relatively greater diameters than the one or more apertures positioned furthest from the channel 110 and the outlet 116.

The urine can also be directed to flow through the second fluid permeable layer 126 and into the channel 110 or outlet 116. A portion of the urine can then flow along the channel 110 and into the outlet 116, as illustrated by flow line 142c. Flow line 142c also illustrates urine flowing from the outlet 116 of the urine collection device 100 to the tube 140, for example, a tube 140 of a suction device (not shown).

**FIG. 3B** is a section view of the urine collection device 100 taken through the section lines 3-3 of **FIG. 1C****.** More specifically, **FIG. 3B** illustrates an example of air flow through the urine collection device 100 when a negative pressure is applied to the interior volume 108 of the receptacle 102. For example, a suction device (*e.g.,* a vacuum source) can generate negative pressure within the urine collection device 100 that draws air in and through the urine collection device 100. Additionally, or alternatively, urine flow through the one or more apertures 118 can generate negative pressure within the interior volume that causes air to be drawn into the interior volume 108 through the air inlet 130.

As shown by the flow line 144a, a negative pressure generated within the interior volume 108 can cause a quantity of air to be drawn into the interior volume 108 through the opening 138 of the air inlet 130. In embodiments, the air can flow into and out of the air inlet 130 substantially uninhibited. In other words, the opening 138 of the air inlet 130 can be a through-hole that is substantially unobstructed such that air can flow into and out of the interior volume 108. In some embodiments, a valve can be disposed within the air inlet 130 (*see* the embodiment shown in **FIGS. 4A** and **4B**) and enable unidirectional or bidirectional air flow relative to the air inlet 130. For example, a unidirectional valve can be disposed within the air inlet 130 that enables air to flow into the interior volume 108 through the air inlet 130 but prevents air from flowing out of the interior volume 108 through the air inlet 130. Conversely, a bidirectional valve can be disposed within the air inlet 130 that enables air to flow into the interior volume 108 through the air inlet 130 and also enables air to flow out of the interior volume 108 through the air inlet 130.

Providing air to the interior volume 108 through the air inlet 130 can lessen or otherwise reduce residual moisture within the interior volume 108. For example, air provided to the interior volume 108 through the air inlet 130 can evaporate residual moisture resultant from an urination event. Reducing or otherwise eliminating moisture within the urine collection device 100 can prevent discomfort to the patient, such as, preventing the formation of a rash or inhibiting an unpleasant smell from emanating from the urine collection device 100.

As illustrated by flow lines 144b, air within the interior volume 108 can be directed through the first fluid permeable layer 124 and through the one or more apertures 118 within the interior layer 106. As illustrated by the flow lines 144b, air can also flow through the second fluid permeable layer 126 and into the channel 110 or outlet 116. A portion of the air can then flow along the channel 110 and into the outlet 116, as illustrated by flow line 144c. Flow line 144c also illustrates air flowing from the outlet 116 of the urine collection device 100 to the tube 140, for example, a tube 140 of a suction device (not shown).

While a negative pressure generated within the device 100 can cause fluid (*e.g.,* urine, air, *etc.*) to flow through the interior layer 106 and out of the outlet 116, a negative pressure is not necessarily required. Rather, generating a positive pressure at the air inlet 130 can additionally or alternatively force fluid (*e.g.,* urine, air, *etc.*) through the urine collection device 100. For example, an air supply (*e.g.,* a source of compressed air) can be coupled to the air inlet 130 to generate a positive pressure within the urine collection device 100 to direct fluid to exit through the outlet 116.

**FIGS. 4A** and **4B** are a section views of the urine collection device 100 taken through section line 3-3 shown in **FIG. 1C****,** according to an embodiment. More specifically, **FIGS. 4A** and **4B** illustrate a unidirectional check valve 146 affixed to the air inlet structure 134 of the air inlet 130. In embodiments, the unidirectional check valve 146 can be an umbrella type check valve which deforms to allow air to pass through the valve when a first side of the valve experiences an increase in pressure (*e.g*., atmospheric pressure) but does not deform to allow air to pass through the valve when a second side of the valve experiences an increase in pressure.

In embodiments, the unidirectional check valve 146 can include a stem 148 and a flexible seal 150. The stem 148 can be coupled to the air inlet structure 134 to removably retain the unidirectional check valve 146 within the air inlet 130. For example, a portion 152 of the stem 148 can be inserted into an aperture 154 within the air inlet structure 134, as illustrated in the embodiment shown in **FIGS. 4A** and **4B****.** In embodiments, the flexible seal 150 can be co-molded or otherwise affixed to the stem 148 such that the flexible seal 150 bisects the opening 138 formed by the air inlet 130. The flexible seal 150 can form a tapered periphery 154 which deflects or bends to provide fluid communication from the first end 136a of the air inlet 130 to the second end 136b of the air inlet 130. In other words, air is permitted to pass through the air inlet 130 and into the interior volume 108 when the flexible seal 150 bends or deforms (as shown in **FIG. 4B**).

While the unidirectional check valve 146 depicted in **FIGS. 4A** and **4B** resembles an umbrella check valve, other types of check valves are contemplated within this disclosure, such as, ball or piston check valves. The unidirectional check valve 146 and the air inlet structure 134 can be disposed at any position between the ends 136a, 136b of the opening 138 of the air inlet 130. For example, the unidirectional check valve 146 and the air inlet structure 134 can be disposed at the second end 136b of the air inlet 130 or the first end 136a of the air inlet 130.

**FIG 4A** illustrates an example of the unidirectional check valve 146 inhibiting or otherwise preventing air or other fluids from exiting the receptacle 102 through the air inlet 130. While in use, fluid like urine may evaporate or otherwise attempt to flow out of the urine collection device 100 through the opening 138 of the air inlet 130, which may cause undesirable consequences (*e.g*., leaks, odor, sanitary issues, etc.). In embodiments, the flexible seal 150 of the unidirectional check valve 146 may block the through-holes 156 within the air inlet structure 134 and thereby prevent urine and/or other fluids (represented by flow lines 158) from exiting the receptacle 102 through the air inlet 130.

**FIG 4B** illustrates an example of the unidirectional check valve 146 enabling or otherwise permitting air or other fluids from entering the receptacle 102 through the air inlet 130. While in use, providing air to the interior volume 108 may be necessary to efficiently extract urine from the receptacle through the outlet 116 (as described in relation to **FIGS. 3A** and **3B**). In embodiments, the flexible seal 150 of the unidirectional check valve 146 may flex or bend to permit air (represented by flow lines 160) to flow through the through-holes 156 and into the interior volume 108.

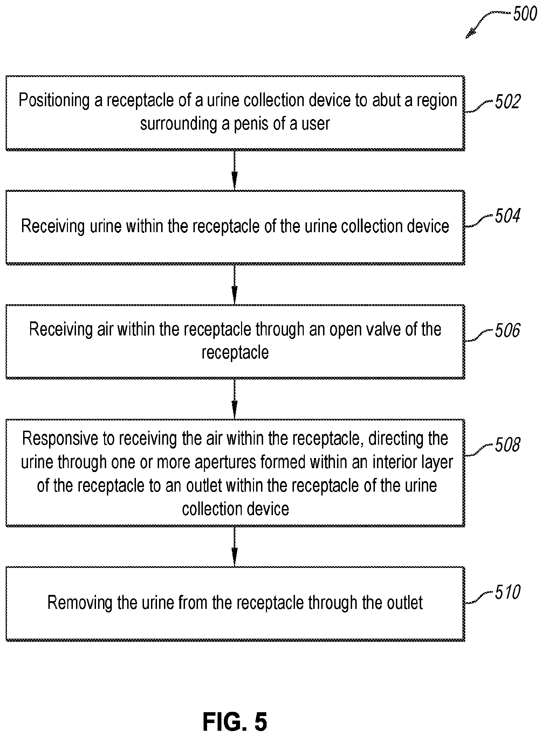

**FIG. 5** is a flowchart illustrating a method 500 of transporting urine away from a body of a user of the urine collection device, according to an embodiment. The method 500 includes the act 502 of positioning a receptacle of a urine collection device to abut a region surrounding a penis of a user. The method 500 includes the act 504 of receiving urine within the receptacle of the urine collection device. The method 500 can include the act 506 of receiving air within the receptacle through a valve of the receptacle. The method 500 can include the act 508 of, responsive to receiving the air within the receptacle, directing the urine through one or more apertures formed within an interior layer of the receptacle to an outlet within the receptacle of the urine collection device. The method 500 includes the act 510 of removing the urine from the receptacle through the outlet. The method 500 can include at least some of acts 502, 504, 506, 508, or 510. The method 500 is for illustrative purposes and, as such, at least one of the acts 502, 504, 506, 508, or 510 can be performed in a different order, split into multiple acts, modified, supplemented, combined, or omitted.

The method 500 includes the act 502 of positioning a receptacle of a urine collection device to abut a region surrounding a penis of a user. The urine collection device can be the same or similar in structure and/or function to any of the urine collection devices described herein. For example, the receptacle can form an interior volume or cavity which receives the penis of the user. The receptacle can form a rim about a periphery of the receptacle that interfaces with the region surrounding the penis of the user. The rim can define a planar surface that receives a material which adheres, affixes, or otherwise removably couples the receptacle to the user. The material, such as hydrogel, can be disposed on the planar surface to create a fluid tight seal or barrier to prevent fluids *(e.g.,* urine, air. *etc.*) within the receptacle from leaking or seeping onto the user from the interior volume of the receptacle.

The method 500 includes the act 504 of receiving urine within the receptacle of the urine collection device. The method 500 can include the act 506 of receiving air within the receptacle through a valve of the receptacle. The method 500 can include the act 508 of, responsive to receiving the air within the receptacle, directing the urine through one or more apertures formed within an interior layer of the receptacle to an outlet within the receptacle of the urine collection device. A negative pressure can be applied to the interior volume when urine is received within the interior volume, for example, the urine can be drawn through the one or more apertures and thereby generate a negative pressure which draws air into the interior volume from the valve of the receptacle.

In embodiments, the quantity, location, and size of the one or more apertures can induce a Venturi effect on the urine wherein urine flowing within the one or more apertures experiences an increased fluid speed and decreased fluid pressure (relative to the fluid pressure of the urine in the interior volume) to draw urine from the interior volume through the one or more apertures. Additionally, or alternatively, the outlet can be in liquid communication with a vacuum pump or suction device which generates negative pressure to draw ambient air into the receptacle through the valve and direct fluid through the one or more apertures. For example, a vacuum tube can be received within the outlet such that urine accumulated within the receptacle can be removed via the vacuum tube.

The method 500 optionally includes positioning a material between the urine collection device and the user. In an embodiment, one or more fluid permeable layers can be positioned between the urine collection device and the user. For example, a first fluid permeable layer can be adhered or otherwise coupled to the interior layer of the receptacle. The first fluid permeable layer can be formed of a wicking material or other material that inhibits moisture from contacting the penis of the user. Additionally or alternatively, a second fluid permeable layer can be adhered or otherwise coupled between the interior layer and an exterior layer of the receptacle. For example, a batting material can be positioned between the interior layer and the exterior layer of the receptacle.

In embodiments, the first fluid permeable layer and/or the second fluid permeable layer can be formed of a material that is urine permeable and has wicking properties. The first and second fluid permeable layers can have a high absorptive rate and a high permeation rate such that urine can be rapidly absorbed and/or transported through the first and second fluid permeable layers. In some embodiments, the first and second fluid permeable layers can be a ribbed knit fabric. In some embodiments, the first and second fluid permeable layers can include and/or have the moisture-wicking characteristic of gauze, felt, terrycloth, thick tissue paper, and/or a paper towel. In some embodiments, the first fluid permeable layer can be soft and/or minimally abrasive such that the first fluid permeable layer does not irritate the skin of the user. The first fluid permeable layer can wick fluid away from the skin of the user such that the moisture within the receptacle is lessened. The second fluid permeable layer can wick fluid away from the interior layer (*e.g.,* the one or more apertures of the interior layer). The wicking properties of the first and second fluid permeable layers can help prevent urine from leaking onto, for example, a hospital bed or a user of the urine collection device. In some embodiments, the first and second fluid permeable layers can be formed of fine denier polyester fibers coated with a thermoplastic water-based binder system.

Some embodiments of the urine collection device can include the first and second fluid permeable layers while other embodiments may include one or none of the first and second fluid permeable layers. For example, an embodiment of the urine collection device can include the first fluid permeable layer coupled to the interior layer and within the interior volume. This embodiment may not include the second fluid permeable layer disposed between the interior layer and the exterior layer of the receptacle.

In embodiments that include the first fluid permeable layer, the first fluid permeable layer can cover at least a portion of an interior surface of the receptacle defined by the interior layer. In an embodiment, the first fluid permeable layer can be coupled to the interior layer such that half of the interior layer is covered by the first fluid permeable layer. For example, the first fluid permeable layer can cover half of the interior layer that is positioned nearest the outlet (*e.g.,* the lower half of the interior layer forming the one or more apertures). In some embodiments, the first fluid permeable layer can be coupled to and cover a substantial majority of the interior surface of the interior layer.

In embodiments that include the second fluid permeable layer, the second fluid permeable layer can cover at least a portion of an interior surface of the exterior layer. In an embodiment, the second fluid permeable layer can be coupled between the interior layer and the exterior layer such that at least the one or more apertures formed by the interior layer are covered by the second fluid permeable layer. For example, the second fluid permeable layer can cover half of the interior surface of the exterior layer nearest the outlet (*e.g.,* the lower half of the exterior layer forming the outlet). In some embodiments, the second fluid permeable layer can be disposed between the interior layer and exterior layer to cover a substantial majority of the interior surface of the exterior layer.

The method 500 includes the act 510 of removing the urine from the receptacle through the outlet. In an embodiment, the receptacle can form at least one channel which directs, funnels, or otherwise guides urine to flow to the outlet. For example, the exterior layer can form a channel that directs urine expelled from the one or more apertures to the outlet. The outlet can define a fluid conduit which enables fluid, such as urine, to flow out of the receptacle. For example, the outlet can be formed within the receptacle at a relatively lower elevation or height when the urine collection device is being worn by a user such that gravity draws urine out of urine collection device through the outlet. Additionally, or alternatively, the outlet can be coupled to a vacuum source which extracts fluids (*e.g.,* urine, air, *etc.*) through the outlet.

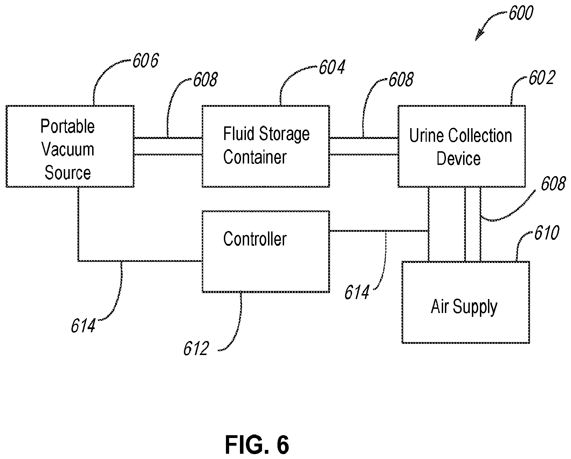

**FIG. 6** is a block diagram of a system 600 for fluid collection, according to an embodiment. The system 600 includes a urine collection device 602, a fluid storage container 604, a portable vacuum source 606, one or more fluid conduits 608, an air supply 610, and a controller 612. The urine collection device 602 may include any of the urine collection devices described herein, such as the urine collection device 100. The urine collection device 602, the fluid storage container 604, and the portable vacuum source 606 may be fluidly coupled to each other via one or more fluid conduits 608. The one or more fluid conduits 608 may include tubes, such as the tube 140 previously described herein. The urine collection device 602 may be operably coupled to one or more of the fluid storage container 604 or the portable vacuum source 606 via the fluid conduit 608. Urine and/or other bodily fluids collected in the urine collection device 602 may be removed from the urine collection device 602 via the fluid conduit 608, which may couple to an outlet of the urine collection device 602. For example, a first open end of the fluid conduit 608 may be coupled to the outlet of the urine collection device 602. The second open end of the fluid conduit 608 may be coupled to the portable vacuum source 606. A suction force may be introduced into an interior volume of the urine collection device 602 via the first open end of the fluid conduit 608 responsive to a suction (e.g., vacuum) force applied at the second end of the fluid conduit 608. The suction force may be applied to the second open end of the conduit 608 by the portable vacuum source 606 either directly or indirectly.

The suction force may be applied indirectly via the fluid storage container 604. For example, the second open end of the fluid conduit 608 may be disposed within the fluid storage container 604 and an additional fluid conduit 608 may extend from the fluid storage container 604 to the portable vacuum source 606. Accordingly, the portable vacuum source 606 may apply suction to the urine collection device 602 via the fluid storage container 604. The suction force may be applied directly via the fluid storage container 604. For example, the second open end of the fluid conduit 608 may be disposed within the portable vacuum source 606. An additional fluid conduit 608 may extend from the portable vacuum source 606 to a point outside of the urine collection device 602, such as to the fluid storage container 604. In such examples, the portable vacuum source 606 may be disposed between the urine collection device 602 and the fluid storage container 604.

In some embodiments, the fluid storage container 604 may include a bag (*e.g.,* drainage bag), a bottle or cup (*e.g.,* collection jar), or any other enclosed container for storing bodily fluids such as urine. In examples, the fluid conduit 608 may extend from the urine collection device 602 and attach to the fluid storage container 604 at a first point therein. An additional fluid conduit 608 may attach to the fluid storage container 604 at a second point thereon and may extend and attach to the portable vacuum source 606. For example, the fluid storage container 604 may include a container fluidly coupled to a first fluid conduit 608 that is also fluidly coupled to the outlet of the urine collection device 602. The container may be fluidly coupled to a second fluid conduit 608 that is also fluidly coupled to a portable vacuum source 606. In such examples, the portable vacuum source 606 may provide a vacuum/suction through the container to the outlet to provide suction in the interior volume of the urine collection device. Accordingly, a vacuum (*e.g*., suction) may be drawn through the urine collection device 602 via the fluid storage container 604. As the fluid (*e.g.,* urine) is drained from the chamber, the fluid may travel through the first section of conduit to the fluid storage container where it may be retained. Fluid, such as urine, may be drained from the urine collection device 602 using the portable vacuum source 606.

The portable vacuum source 606 may include one or more of a manual vacuum pump, and electric vacuum pump, a diaphragm pump, a centrifugal pump, a displacement pump, a magnetically driven pump, a peristaltic pump, or any pump configured to produce a vacuum. The portable vacuum source 606 may provide a vacuum or suction to remove fluid from the outlet of the urine collection device 602. In some embodiments, the portable vacuum source 606 may be powered by one or more of a power cord (*e.g.,* connected to a power socket), one or more batteries, or even manual power (*e.g.,* a hand operated vacuum pump). In examples, the portable vacuum source 606 may be sized and shaped to fit outside of, on, or within the urine collection device 602. For example, the portable vacuum source 606 may include one or more miniaturized pumps or one or more micro pumps. The portable vacuum sources 606 disclosed herein may include one or more of a switch, a button, a plug, a remote, or any other device suitable to activate the portable vacuum source 606. It should be understood that the portable vacuum sources 606 disclosed herein may provide a portable means of providing a suction or vacuum that allows use of the devices and systems herein outside of hospital or care facility environments where vacuum lines are plumbed into patient rooms or large (*e.g.,* larger or heavier than a patient can readily carry) vacuum sources are located. For example, a portable vacuum source may be small and light enough to be carried by a user (*e.g.,* patient) or aid (*e.g.,* nurse) during transportation of the user.

The air supply 610 can provide air to an air inlet of the urine collection device 602. The air supply 610 can be a stand-alone device or an air source built within a structure (*e.g.,* an air supply line within a hospital room). The urine collection device 602 can be coupled to the air supply 610 via a fluid conduit 608. For example, a first end of the fluid conduit 608 can be coupled to the air inlet of the urine collection device 602 while a second end of the fluid conduit 608 can be coupled to the air supply 610. As previously described herein, the air supply 610 can provide air to the interior volume of the receptacle to evaporate or otherwise inhibit the presence of humidity or moisture within the interior volume of the receptacle. Additionally, or alternatively, the air supplied to the interior volume can generate a positive pressure within the receptacle that drives air and other fluids from the interior volume and into the outlet of the urine collection device 602.

The controller 612 can be communicatively coupled (via electrical connections 614) to one or more of the urine collection device 602, portable vacuum source 606, or the air supply 610. The controller 612 can direct the portable vacuum source 606 and/or the air supply 610 to operate when a moisture event is detected. In any of the examples, systems, or devices disclosed herein, the urine collection device 602 may include moisture sensors (not shown) disposed inside of the urine collection device 602. In such examples, the moisture sensor may be operably coupled to the controller 612 or directly to the portable vacuum source 606, and may provide electrical signals indicating that moisture is or is not detected within the urine collection device 602. The moisture sensor(s) may provide an indication that moisture is present, and responsive thereto, the controller 612 or portable vacuum source 606 may direct the initiation of suction to the outlet to remove the fluid from the urine collection device 602. Suitable moisture sensors may include capacitance sensors, volumetric sensors, potential sensors, resistance sensors, frequency domain reflectometry sensors, time domain reflectometry sensors, or any other suitable moisture sensor. In practice, the moisture sensors may detect moisture in the urine collection device 602 and may provide a signal to the controller 612 and/or portable vacuum source 606 to activate the portable vacuum source 606. In other embodiments, the controller 612 may direct the portable vacuum source 606 and/or the air supply 610 to continuously operate.

## Claims

1. A urine collection device (100), comprising:
a receptacle (102) defining an interior volume (108) and configured to receive a penis of a user;
an air inlet (130) in fluid communication with the interior volume, the air inlet being at least partially defined by the receptacle; and
an outlet (116) in fluid communication with the interior volume, the outlet being formed within the receptacle and configured to enable extraction of fluid from the receptacle:
wherein the receptacle includes a first layer (106) and a second layer (104) affixed to the first layer, **characterised in that** the second layer defines one or more channels (110) which direct fluid to the outlet;
and wherein the first layer defines one or more apertures through which urine can exit the interior volume; and
the second layer is fluid impermeable
whereby air from the air inlet and urine from the penis of the user can flow from the receptacle through the aperture(s) and along the channel(s) to the outlet.

2. The urine collection device of claim 1, further comprising a fluid permeable layer positioned within the interior volume, the fluid permeable layer overlaying the one or more apertures.

3. The urine collection device of claim 2, wherein the fluid permeable layer is retained within the interior volume by an adhesive.

4. The urine collection device of claim 1, 2 or 3, further comprising a batting layer disposed between at least a portion of the first and second layers.

5. The urine collection device of any one of the preceding claims, wherein the outlet is configured to receive and retain a tube of a suction device.

6. The urine collection device of any one of the preceding claims, wherein the receptacle forms a rim surrounding a periphery of the receptacle, the rim having a shape that interfaces with a region surrounding the penis of a user.

7. The urine collection device of any one of the preceding claims, wherein the air inlet includes a valve.

8. A system for transporting urine away from a penis of a user, the system comprising:
a urine collection device as claimed in any one of the preceding claims and
a suction device coupled to the outlet.

## Patentansprüche

1. Urinsammelvorrichtung (100), umfassend:
einen Behälter (102), der ein Innenvolumen (108) definiert und ausgebildet ist, um einen Penis eines Benutzers aufzunehmen;
einen Lufteinlass (130), der in Fluidverbindung mit dem Innenvolumen steht, wobei der Lufteinlass zumindest teilweise durch den Behälter definiert ist; und
einen Auslass (116), der in Fluidverbindung mit dem Innenvolumen steht, wobei der Auslass innerhalb des Behälters gebildet ist und ausgebildet ist, um eine Entnahme von Fluid aus dem Behälter zu ermöglichen:
wobei der Behälter eine erste Schicht (106) und eine zweite Schicht (104), die an der ersten Schicht befestigt ist, einschließt, **dadurch gekennzeichnet, dass** die zweite Schicht einen oder mehrere Kanäle (110) definiert, die Fluid zum Auslass leiten;
und wobei die erste Schicht eine oder mehrere Öffnungen definiert, durch die Urin aus dem Innenvolumen austreten kann; und
die zweite Schicht fluidundurchlässig ist,
wodurch Luft aus dem Lufteinlass und Urin aus dem Penis des Benutzers vom Behälter durch die eine oder die mehreren Öffnungen und entlang des einen oder der mehreren Kanäle zum Auslass strömen können.

2. Urinsammelvorrichtung nach Anspruch 1, weiter umfassend eine fluiddurchlässige Schicht, die innerhalb des Innenvolumens positioniert ist, wobei die fluiddurchlässige Schicht die eine oder die mehreren Öffnungen überlagert.

3. Urinsammelvorrichtung nach Anspruch 2, wobei die fluiddurchlässige Schicht durch einen Klebstoff innerhalb des Innenvolumens gehalten wird.

4. Urinsammelvorrichtung nach Anspruch 1, 2 oder 3, weiter umfassend eine Watteschicht, die zwischen zumindest einem Abschnitt der ersten und der zweiten Schicht angeordnet ist.

5. Urinsammelvorrichtung nach einem der vorstehenden Ansprüche, wobei der Auslass ausgebildet ist, um einen Schlauch einer Saugvorrichtung aufzunehmen und zu halten.

6. Urinsammelvorrichtung nach einem der vorstehenden Ansprüche, wobei der Behälter einen Rand bildet, der einen Umfang des Behälters umgibt, wobei der Rand eine Form aufweist, die an eine Region ankoppelt, die den Penis eines Benutzers umgibt.

7. Urinsammelvorrichtung nach einem der vorstehenden Ansprüche, wobei der Lufteinlass ein Ventil einschließt.

8. System zum Abtransportieren von Urin vom Penis eines Benutzers, das System umfassend:
eine Urinsammelvorrichtung nach einem der vorstehenden Ansprüche und
eine Saugvorrichtung, die an den Auslass gekoppelt ist.

## Revendications

1. Dispositif (100) de collecte d'urine, comprenant :
un réceptacle (102) définissant un volume intérieur (108) et configuré pour recevoir un pénis d'un utilisateur ;
une entrée d'air (130) en communication fluidique avec le volume intérieur, l'entrée d'air étant au moins partiellement définie par le réceptacle ; et
une sortie (116) en communication fluidique avec le volume intérieur, la sortie étant formée à l'intérieur du réceptacle et configurée pour permettre l'extraction de fluide du réceptacle :
dans lequel le réceptacle inclut une première couche (106) et une seconde couche (104) fixée à la première couche, **caractérisé en ce que** la seconde couche définit un ou plusieurs canaux (110) qui dirigent le fluide vers la sortie ;
et dans lequel la première couche définit une ou plusieurs ouvertures à travers lesquelles l'urine peut sortir du volume intérieur ; et
la seconde couche est imperméable aux fluides
moyennant quoi de l'air provenant de l'entrée d'air et de l'urine provenant du pénis de l'utilisateur peuvent s'écouler du réceptacle à travers la/les ouverture(s) et le long du/des canal/canaux jusqu'à la sortie.

2. Dispositif de collecte d'urine selon la revendication 1, comprenant en outre une couche perméable aux fluides positionnée à l'intérieur du volume intérieur, la couche perméable aux fluides recouvrant les une ou plusieurs ouvertures.

3. Dispositif de collecte d'urine selon la revendication 2, dans lequel la couche perméable aux fluides est retenue à l'intérieur du volume intérieur par un adhésif.

4. Dispositif de collecte d'urine selon la revendication 1, 2 ou 3, comprenant en outre une couche ouatée disposée entre au moins une partie des première et seconde couches.

5. Dispositif de collecte d'urine selon l'une quelconque des revendications précédentes, dans lequel la sortie est configurée pour recevoir et retenir un tube d'un dispositif d'aspiration.

6. Dispositif de collecte d'urine selon l'une quelconque des revendications précédentes, dans lequel le réceptacle forme un rebord entourant une périphérie du réceptacle, le rebord ayant une forme qui s'interface avec une région entourant le pénis d'un utilisateur.

7. Dispositif de collecte d'urine selon l'une quelconque des revendications précédentes, dans lequel l'entrée d'air inclut une valve.

8. Système de transport d'urine à distance d'un pénis d'un utilisateur, le système comprenant :
un dispositif de collecte d'urine selon l'une quelconque des revendications précédentes et
un dispositif d'aspiration accouplé à la sortie.
